(19) Europäisches Patentamt
European Patent Office
Office européen des brevets

(11) Veröffentlichungsnummer : **0 222 693 B1**

# (12) EUROPÄISCHE PATENTSCHRIFT

(45) Veröffentlichungstag der Patentschrift : **17.04.91 Patentblatt 91/16**

(21) Anmeldenummer : **86730187.1**

(22) Anmeldetag : **11.11.86**

(51) Int. Cl.$^5$ : **C07D 471/04,** A61K 31/435, // (C07D471/04, 221:00, 209:00)

## (54) Neue 3-Oxadiazol- und 3-Carbonsäure-beta-Carbolinderivate, ihre Herstellung und ihre Verwendung als Arzneimittel.

(30) Priorität : **13.11.85 DE 3540653**

(43) Veröffentlichungstag der Anmeldung : **20.05.87 Patentblatt 87/21**

(45) Bekanntmachung des Hinweises auf die Patenterteilung : **17.04.91 Patentblatt 91/16**

(84) Benannte Vertragsstaaten : **AT BE CH DE ES FR GB GR IT LI LU NL SE**

(56) Entgegenhaltungen :
**EP-A- 0 110 813**
**EP-A- 0 137 390**
**EP-A- 0 161 574**
**EP-A- 0 161 575**
**DE-A- 3 504 045**
**PATENT ABSTRACTS OF JAPAN, unexamined applications, Field C, Band 6, Nr. 63, 22. April 1982 THE PATENT OFFICE JAPANESE GOVERNMENT Seite 138 C 99**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Huth, Andreas, Dr.**
**Ermannstrasse 19**
**W-1000 Berlin 41 (DE)**
Erfinder : **Schmiechen, Ralph, Dr.**
**Bayernring 27**
**W-1000 Berlin 42 (DE)**
Erfinder : **Stephens, David Norman, Dr.**
**Hildegardstrasse 16a**
**W-1000 Berlin 31 (DE)**
Erfinder : **Engelstoft, Mogens, Dr.**
**Mosegard Park 121**
**DK-3500 Vaerlose (DK)**
Erfinder : **Wätjen, Frank, Dr.**
**Jostiens Vej 27**
**DK-2880 Bajsvaerd (DK)**
Erfinder : **Hansen, John Bondo, Dr.**
**Harretoften 10**
**DK-2800 Lyngby (DK)**
Erfinder : **Jensen, Leif Helth, Dr.**
**Nordkrog 19**
**DK-2900 Hellerup (DK)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die Erfindung betrifft neue 3-Oxadiazol- und 3-Carbonsäureester-β-carbolinderivate, ihre Herstellung und ihre Verwendung als Arzneimittel.

Die erfindungsgemäßen Verbindungen besitzen wertvolle pharmakologische Eigenschaften. Sie beeinflussen insbesondere das Zentralnervensystem und eignen sich somit als Psychopharmaka.

Die erfindungsgemäßen Verbindungen haben die allgemeine Formel I

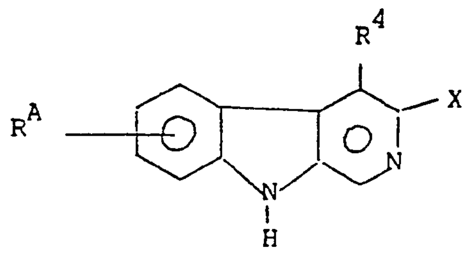

(I)

worin

X eine $COOR^3$-Gruppe mit $R^3$ in der Bedeutung von niederem Alkyl ist oder einen Oxadiazolylrest der Formel

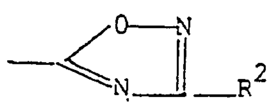

oder

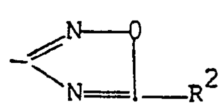

mit $R^2$ in der Bedeutung von H, niederem Alkyl oder Cycloalkyl darstellt und

$R^4$ Wasserstoff, niederes Alkyl oder niederes Alkoxyalkyl bedeutet und

$R^A$ eine CH $R^1ZR^5$-Gruppe ist, worin Z Schwefel oder Sauerstoff bedeutet,

$R^1$ niederes Alkyl oder gegebenenfalls substituiertes Phenyl ist und

$R^5$ Wasserstoff, Phenyl oder gegebenenfalls substituiertes niederes Alkyl ist und jede Verbindung 1 oder 2 $R^A$-Reste enthalten kann, wobei $R^3$ nicht Ethyl ist, wenn $R^4$ $CH_3$ und $R^A$ 5-CH $(OC_2H_5)$ $C_2H_5$ und wenn $R^4$ $CH_2OCH_3$ und $R^A$ 5-CH$(OC_2H_5)$-$CH_3$ bedeuten.

Die neuen –β-Carbolinderivate der allgemeinen Formel I können im A-Ring in Position 5-8 ein- oder zweifach substituiert sein, wobei die Substitution in 5- oder 6-Stellung bevorzugt ist.

Unter niederem Alkyl sind sowohl gerad- als auch verzweigtkettige Reste mit $C_1$-$C_6$ Kohlenstoffatomen zu verstehen. Beispielsweise seien Alkylreste genannt wie Methyl, Ethyl, n-Propyl, iso-Propyl, n-Butyl, iso-Butyl, tert.-Butyl, sek. Butyl, Pentyl, Hexyl.

Der Cycloalkylrest $R^2$ enthält 3-7 Kohlenstoffatome, wobei als bevorzugt Reste mit 3-5 Kohlenstoffatomen genannt seien wie beispielsweise Cyclopropyl, Methylcyclopropyl, Cyclobutyl, Cyclopentyl.

Der niedere Alkylrest $R^5$ kann substituiert sein durch ein oder mehrere Halogene wie beispielsweise Fluor, Chlor u.a. oder $C_{1-2}$-Alkoxygruppen oder Phenyl.

Geeignete Substituenten für Phenyl sind beispielsweise Halogene wie Fluor, Chlor, Brom, Jod und niedere Alkyl- und Alkoxy-Gruppen, die 1-3fach in jeder Position des Aromaten stehen können.

Die erfindungsgemäßen Verbindungen zeigen überraschenderweise im Vergleich zu den in der Europäischen Patentanmeldung Nr. 54507 beschriebenen am A-Ring nicht verzweigten β-Carbolinen im pharmakologischen Test überlegene psychotrope Eigenschaften wie aus der Tabelle 1 am Beispiel einiger erfindungsgemäßer Verbindungen zu ersehen ist.

TABELLE 1

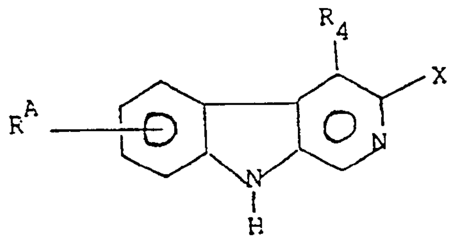

| $R_A$ | $R_4$ | X | $IC_{50}$ ng/ml in vitro | $ED_{50}$ mg/kg in vivo | PTZ $ED_{50}$(mg/kg) |
|---|---|---|---|---|---|
| $5-CH_2OC_2H_5$ | $CH_3$ | $COOC_2H_5$ | 0.8 | 1.0 | > 100 |
| $5-CH_2O\ CH_3$ | $CH_3$ | $COOC_2H_5$ | 0.45 | 2.0 | > 100 |
| $5-CH(CH_3)OC_3H_7$ | $CH_3$ | $COOC_2H_5$ | 0.4 | 0.6 | 1 |
| $5-CH(CH_3)OC_3H_7$ | $CH_3$ | COO 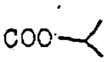 | 0.75 | 1.8 | 0.8 |
| $5-CH(CH_3)OC_2H_5$ | $CH_3$ | 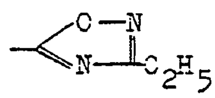 | 0.3 | 0.09 | 0.4 |
| $5-CH(C_2H_5)OC_2H_5$ | $CH_3$ | 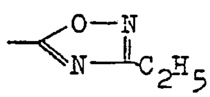 | 0.4 | 3 | 0.6 |
| $5-CH(C_2H_5)OC_2H_5$ | $CH_2OCH_3$ | 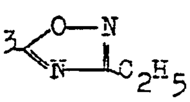 | 0.2 | 3 | 0.3 |
| $6-CH(CH_3)OC_2H_5$ | $C_2H_5$ | 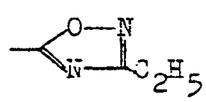 | 0.7 | 1.1 | 2.3 |
| $6-CH(CH_3)OCH_2CF_3$ | $CH_3$ | $COOC_2H_5$ | 0.8 | 1.8 | 2.4 |
| $6-CH(CH_3)SC_2H_5$ | $CH_3$ | $COOC_2H_5$ | 1.2 | 0.43 | 4 |

Es ist bekannt, daß bestimmte Stellen im zentralen Nervensystem von Vertebraten eine hohe spezifische Affinität für die Bindung von 1.4 und 1.5- Benzodiazepinen aufweisen (Squires, R.F. und Braestrup, C., Nature (London) 266 (1977), 734). diese Stellen werden Benzodiazepin-Rezeptoren genannt.

Die pharmakolgischen Eigenschaften der erfidungsgemäßen Verbindungen wurden durch Untersuchung

des Verdrängungsvermögens von radioaktiv-markiertem Flunitrazepam von Benzodiazepin-Rezeptoren bestimmt.

Die Verdrängungsaktivität der erfindungsgemäßen Verbindungen wird als $IC_{50}$- und $ED_{50}$-Wert angegeben. Der $IC_{50}$-Wert gibt die Konzentration an, die eine 50%ige Verdrängung der spezifischen Bindung von $H^3$-Flunitrazepam (1,0 nM, 0°C) in Proben mit einem Gesamtvolumen von 0, 55 ml einer Suspension von Hirnmembranen, z.B. von Ratten bewirkt.

Der Verdrängungstest wird wie folgt ausgeführt :

0,5 ml einer Suspension von unbehandeltem Rattenvorderhirn in 25 mM $KH_2PO_4$, pH = 7,1 (5-10 mg Gewebe/Probe) wird für 40 bis 60 Minuten bei 0°C zusammen mit $^3H$-Diazepam (spezifische Aktivität 14,4 Ci/mmol, 1,9 nM) oder $^3H$-Flunitrazepam (spezifische Aktivität 87 Ci/mmol, 1,0 nM) inkubiert. Nach Inkubation wird die Suspension durch eine Glasfritte filtriert, der Rückstand zweimal mit kalter Pufferlösung gewaschen und die Radioaktivität im Scintillationszähler gemessen.

Der Versuch wird dann wiederholt, jedoch so, daß vor der Zugabe des radioaktiv-markiertem Benzodiazepins eine bestimmte Menge oder eine überschüssige Menge der Verbindung, deren Verdrängungsaktivität zu bestimmen ist, zugesetzt wird. Auf Grundlage der erhaltenen Werte kann dann der $IC_{50}$- Wert berechnet werden.

Der $ED_{50}$-Wert stellt die Dosis einer Versuchssubstanz dar, die eine Reduktion der spezifischen Bindung des Flunitrazepams an dem Benzodiazepin-Rezeptor in einem lebenden Gehirn auf 50% des Kontrollwertes bewirkt.

Der in-vivo Test wird wie folgt ausgeführt :

Gruppen von Mäusen wird die Versuchssubstanz bei unterschiedlichen Dosen und normalerweise intraperitoneal injiziert. Nach 15 Minuten wird den Mäusen das $^3H$-Flunitrazepam intravenös verabreicht. Nach weiteren 20 Minuten werden die Mäuse getötet ihr Vorderhirn entfernt und die an die Hirnmembranen spezifisch gebundene Radioaktivität durch Scintillationszählung gemessen. Der $ED_{50}$-Wert wird aus den Dosis/Wirkungs-Kurven bestimmt.

Die erfindungsgemäßen Verbindungen zeigen im pharmakologischen Test insbesondere anxiolytische und antikonvulsive Wirksamkeit. Zur Untersuchung der antikonvulsiven Wirkung wurde die Aufhebung der mit Pentylentetrazol (Pentazol) induzierten Krämpfe untersucht. Pentazol wird in einer Menge von 150 mg/kg als Salzsäure-Lösung (pH 2-3) subcutan 15-30 Minuten nach der intraperitonealen Applikation der Testsubstanz gegeben. Diese Menge induziert klonische und tonische Krämpfe, die bei unbehandelten Tieren zum Tode führen. Die Zahl der Mäuse, die Krämpfe zeigen und die Zahl derer, die 30 Minuten nach Pentazol gestorben sind, wird registriert.

Die in der Tabelle angegebenen $ED_{50}$-Werte wurden nach der Methode von Litchfield und Wilcoxon (J. Pharmacol. exp. Ther. 96 (1949) 99-103) als die Menge der antagonistisch wirkenden Substanz bestimmt, die 50% der Tiere vor Krämpfen und Tod schützt.

Die neuen Verbindungen der allgemeinen Formel I besitzen wertvolle pharmakologische Eigenschaften. Insbesondere wirken sie sich auf das Zentralnervensystem aus und sind somit als Psychopharmaka für die Humanmedizin geeignet. Die Verbindungen können besonders zur Behandlung von Angst begleitet von Depressionen, Epilepsie, Schlafstörungen, Spastizitäten und Muskelrelaxation während der Anästhesie eingesetzt werden. Die erfindungsgemäßen Verbindungen zeigen auch amnestische bzw. gedächtnisfördernde Eigenschaften.

Die erfindungsgemäßen Verbindungen können zur Formulierung von pharmazeutischen Präparationen, zum Beispiel für die orale und parenterale Anwendung bei Säugetieren einschließlich Menschen, nach an sich bekannten Methoden der Galenik verwendet werden.

Als Hilfsstoffe zur Formulierung von pharmazeutischen Präparationen sind solche physiologisch verträglichen organischen und anorganischen Trägersubstanzen für die enterale und parenterale Anwendung geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermitteln, Konservierunsstoffen, Stabilisatoren, Netzmitteln, Emulgatoren, Puffermittel und Farbstoffen, versetzt werden.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Für die orale Anwendung sind insbesondere Tabletten, Dragees oder Kapseln mit Talkum und/oder einem Kohlenwasserstoffträger oder -binder, wie zum beispiel Lactose, Mais- oder Kartoffelstärke, geeignet. Die Anwendung kann auch in flüsssiger Form erfolgen, wie zum Beispiel als Saft, dem gegebenenfalls ein Süßstoff

beigefügt wird.

Die erfindungsgemäßen Verbindungen werden in einer Dosiseinheit von 0,05 bis 100 mg aktiver Substanz in einem physiologisch verträglichen Träger eingebracht.

Die erfidugnsgemäßen Verbindungen werden in einer Dosis von 0,1 bis 300 mg/Tag, vorzugsweise 1-30 mg/Tag, angewendet.

Die Herstellung der erfindungsgemäßen Verbindungen der allgemeinen Formel I erfolgt nach an sich bekannten Methoden. Beispielsweise erfolgt die Herstellung der Verbindung der allgemeinen Formel I, dadurch, daß man

a) eine Verbindung der allgemeinen Formel II

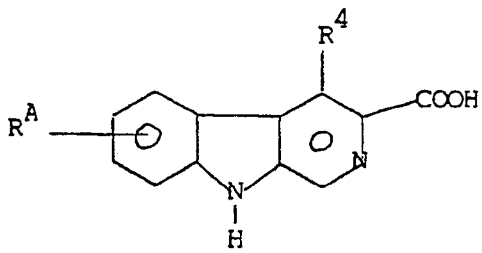

(II)

worin $R^A$ und $R^4$ die oben genannte Bedeutung haben mit einer Verbindung der Formel

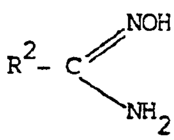

worin $R^2$ die oben genannte Bedeutung hat, umsetzt zu einer Verbindung der allgemeinen Formel I, in der X für den Rest

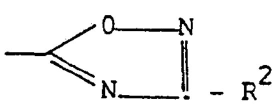

mit $R^2$ in der oben genannten Bedeutung steht,

b) eine Verbindung der allgemeinen Formel III

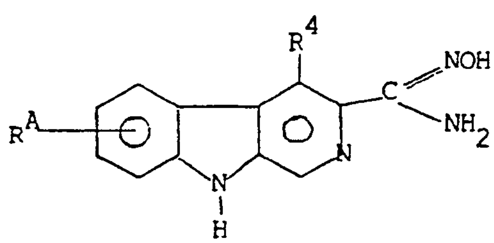

(III),

worin

$R^A$ und $R^4$ die oben genannte Bedeutung haben,

mit einem Carbonsäureanhydrid $(R^2CO)_2$ 0, worin $R^2$ die oben genannte Bedeutung hat, umwetzt zu einer Verbindung der allgemeinen Formel I, in der X für den Rest

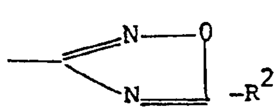

mit $R^2$ in der oben genannten Bedeutung steht,

c) eine Verbindung der allgemeinen Formel IV

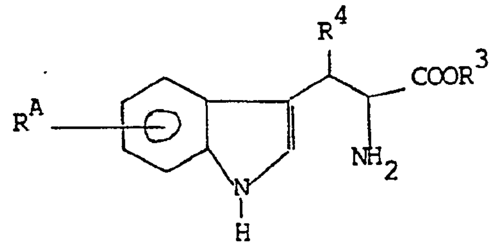

(IV)

worin

$R^4$ die oben genannte Bedeutung hat,

$R^A$ Wasserstoff oder die oben genannten Reste bedeutet und

$R^3$ Methyl oder Ethyl ist,

cyclisiert und aromatisiert und, falls $R^A$ Wasserstoff ist, acyliert zum 6-Acyl-Derivat und dieses reduziert zu einer Verbindung der allgemeinen Formel I mit $R^A$ in der Bedeutung von $CHR^1$ OH, worin $R^1$ die obige Bedeutung hat und anschließend gegebenenfalls eine freie Hydroxygruppe verethert und/ oder eine Estergruppe umestert.

Zur Einführung des 1,2,4-Oxadiazol-5-yl-Restes wird die β-Carbolincarbonsäure der allgemeinen Formel II mit einem Amidoxim der Formel

$$R^2\text{-}C(=NOH)NH_2,$$

in einem inerten Lösungsmittel, das über 100°C siedet und gegenüber dem Reaktanten inert ist, bei der Rückflußtemperatur des Reaktionsgemisches zur Kondensation gebracht. Geeignete Lösungsmittel für die Kondensationsreaktion sind beispielsweise Toluol und Dimethylformamid. Zweckmäßigerweise wird die freie β-Carbolin-3-carbonsäure vor der Kondensationsreaktion in geeigneter Weise aktiviert. Hierzu kann die freie Säure z.B. in das gemischte Anhydrid, in den aktivierten Ester oder in das Chlorid überführt werden.

Gut bewährt hat sich auch eine Aktivierung zum Imidazolid mit Imidazol/Thionylchlorid (oder auch Carbonyldiimidazol) in einem aprotischen Lösungsmittel wie Dioxan, Tetrahydrofuran, Dimethylformamid oder N-Methylpyrrolidon bei Temperaturen zwischen 0 und 50°C, vorzugsweise Raumtemperatur.

Zur Einführung des 1,2,4-Oxadiazol-3-yl-Restes setzt man beispielsweise das 3-Carbonsäurenitril mit Hydroxylamin zu der Verbindung der allgemeinen Formel III um. Das so erhaltene β-Carbolin-3-carboxamidoxim wird bei Raumtemperatur mit dem Säureanhydrid $(R^2CO)_2O$ versetzt und anschließend bis zur Siedetemperatur erwärmt. Die Reaktion ist nach ca. 7 Stunden beendet und wird nach dem üblichen Verfahren aufgearbeitet.

Die Cyclisierung nach Verfahrensvariante c) wird vorgenommen, indem man die Verbindung der allgemeinen Formel IV in einem inerten mit Wasser nicht mischbaren Lösungsmittel wie Benzol, Toluol, Xylol, Chlorbenzol, Anisol, Mesitylen löst und mit Paraformaldehyd gegebenenfalls bei erhöhter Temperatur umsetzt. Die Cyclisierung kann auch mit Glyoxylsäure erfolgen. Hierbei versetzt man zweckmäßigerweise das Amin, das in Wasser oder in einem organischen Lösungsmittel wie beispielsweise Essigester gelöst ist, mit einer wässrigen Lösung von Glyoxylsäure bei einem pH-Wert von 0-7, vorzugsweise 4. Die Decarboxylierung wird bei erhölter Tenperatur, gegebenenfalls bei der Siedetemperatur eines oben genannten inerten Lösungsmittels, zum Beispiel Toluol oder Xylol, vorgenommen.

Bei der Cyclisierung entsteht ein 1.2.3.4-Tetrahydro-9H-Pyrido-[3.4-b]-indolderivat, das anschließend in beiden Fällen dehydriert wird.

Die Dehydrierung kann beispielsweise so durchgeführt werden, daß man das Ausgangsmaterial in einem inerten Lösungsmittel löst bzw. suspendiert und elementaren Schwefel hinzugibt, dessen Menge in etwa so bemessen ist, daß pro Doppelbindung ein Moläquivalent Schwefel verwendet wird. Das Reaktionsgemisch wird mehrere Stunden am Rückfluß gekocht, wobei der Reaktionsverlauf dünnschichtchromatographisch verfolgt wird. Alle aprotischen Lösungsmittel, deren Siedepunkt über 100°C liegt und die gegenüber dem Ausgangsmaterial inert sind, wie beispielsweise Xylol, Mesitylen, Anisol, Toluol, Chlorbenzol und Diphenyläther sind für die Dehydrierung geeignet.

Eine weitere Methode ist die Dehydrierung mit Edelmetallkatalysatoren wie Platin in feinverteilter Form, Palladium-Mohr oder Palladium-Kohle in Xylol, Mesitylen oder Cumol bei 120-180°C und Reaktionszeiten von 2-6 Stunden.

Eine andere Methode ist die Dehydrierung mit tert.-Butylhypochlorit und tertiären Basen. (Deutsche Patentanmeldung Nr. 3 504 045.9)

Wird eine Umesterung gewünscht, so kann man zum Beispiel mit dem entsprechenden Alkohol oder Alkalialkoholat umsetzen, gegebenenfalls kann man Titantetra-isopropylat als Katalysator im wasserfreien Alkohol zufügen. Üblicherweise wird die Umesterung bei Temperaturen von 60-120°C durchgeführt und ist nach ca. 2-6 Stunden beendet.

Die Einführung der tert.-Butylestergruppe erfolgt beispielsweise durch Umsetzung der Carbonsäure mit tert.-Butoxy-bis-dimethylaminomethan. Im allgemeinen wird die Reaktion unter Inertgasatmosphäre wie Argon oder Stickstoff und unter Feuchtigkeitsausschluß bei erhöhter Temperatur durchgeführt.

Die Ester können auch über Aktivierung der entsprechenden Säure und anschließende Umsetzung mit dem gewünschten Alkohol hergestellt werden.

Aliphatische Hydroxygruppen werden in einem inerten Lösungsmittel beispielsweise Methylenchlorid, Tetrahydrofuran u.a. mit einem Alkylhalogenid wie zum Beispiel Chlorid, Bromid oder Jodid oder einem Alkyltosylat in Gegenwart von Tetrabutylammoniumhydrogensulfat und gepulverter KOH verethert. Auch eine Umsetzung der Hydroxyverbindung mit Thionylchlorid bei Temperaturen von −10°C bis + 30°C und anschließender Behandlung mit Alkohol ist möglich.

6-Acyl-Derivate können beispielsweise unter Friedel-Crafts-Bedingungen durch Umsetzung mit Säurechloriden in Gegenwart von Lewis-Katalysatoren erhalten werden.

Die Säurechloride leiten sich bevorzugt von aliphatischen $C_{2-4}$-Carbonsäuren ab wie beispielsweise Essigsäure, Propionsäure, Buttersäure, u.a. und aromatischen Carbonsäuren wie beispielsweise Benzoesäure.

Die so erhaltenen Ketone können mit den üblichen Reduktionsmitteln wie beispielsweise $NaBH_4$ in die entsprechenden Alkohole überführt werden.

Die Herstellung der Ausgangsverbindungen ist bekannt oder kann nach bekannten Verfahren erfolgen.

So kann die Verseifung der Estergruppe sauer oder alkalisch erfolgen ; vorzugsweise wird alkalisch verseift, indem Ester mit verdünnter wässriger Alkalilauge wie Kalium- oder Natriumhydroxid, in einem protischen Lösungsmittel, wie zum Beispiel Methanol, Ethanol oder Ethylenglykol, auf Temperaturen bis zur Rückflußtemperatur des Reaktionsgemisches erhitzt wird.

Carboxamidoxime lassen sich in bekannter Weise aus den β-Carbolincarbonsäuren herstellen. Die wie üblich dargestellten Säureamide können mit wasserabspaltenden Mitteln wie zum Beispiel einem Reagenz aus Triphenylphosphin/Brom in Gegenwart von Triethylamin in die entsprechenden Nitrile überführt werden. Diese können anschließend mit Hydroxylamin zu den gewünschten Carboxamidoximen umgesetzt werden.

Die nachfolgenden Beispiele sollen die erfindungsgemäßen Verfahren erläutern.

Die benötigten Carbonsäuren wurden wie folgt hergestellt :

500 mg 5-(1-Äthoxyäthyl)-4-methyl-β-carbolin-3-carbonsäureäthylester wurden mit 5 ml 1n-Natronlauge und 20 ml Äthanol 3 Stunden am Rückfluß gekocht. Anschließend wurde mit Eisessig sauer gestellt, abgesaugt und mit Wasser gewaschen. Man erhielt 400 mg 5-(1-Äthoxyäthyl)-4-methyl-β-carbolin-3-carbonsäure, die nach gutem Trocknen über Phosphorpentoxid am Vakuum weiter umgesetzt wurde.

In analoger Weise wurden hergestellt :

6-(1-Hydroxyethyl)-β-carbolin-3-carbonsäure
6-(1-Hydroxyethyl)-4-methyl-β-carbolin-3-carbonsäure
6-(1-Methoxyethyl)-4-methyl-β-carbolin-3-carbonsäure
6-(1-Äthoxyethyl)-4-methyl-β-carbolin-3-carbonsäure
6-[1-(2,2,2-Trifluorethoxy)-ethyl]-4-methyl-β-carbolin-3-carbonsäure
6-(1-Isopropoxyethyl)-β-carbolin-3-carbonsäure
6-(1-Ethylthioethyl)-4-methyl-β-carbolin-3-carbonsäure
6-(1-Ethoxyethyl)-4-ethyl-β-carbolin-3-carbonsäure
6-(1-Butoxyethyl)-β-carbolin-3-carbonsäure
5-(1-Ethoxypropyl)-4-methyl-β-carbolin-3-carbonsäure
5-(1-Ethoxyethyl)-4-methoxymethyl-β-carbolin-3-carbonsäure
6-(1-Propoxyethyl)-β-carbolin-3-carbonsäure
5-(1-Methoxyethyl)-4-ethyl-β-carbolin-3-carbonsäure
6-(1-Methoxyethyl)-β-carbolin-3-carbonsäure
6-(1-Hydroxybenzyl)-β-carbolin-3-carbonsäure
6(1-Hydroxyethyl)-4-ethyl-β-carbolin-3-carbonsäure 6-(1-Phenoxyethyl)-4-methyl-β-carbolin-3-carbonsäure
5-(1-Ethoxypropyl)-4-methoxymethyl-β-carbolin-3-carbonsäure

## BEISPIEL 1

### 5-(1-Ethoxyäthyl)-4-methyl-β-carbolin-3-carbonsäureethylester

A. 25 g (83 mMol) 1-Tosyl-4-hydroxymethylindol in 600 ml Methylenchlorid werden unter Rühren und Kühlen portionsweise mit 72,5 g (830 mMol) Mangan-IV-oxid versetzt. Nach 4 Stunden Rührzeit wird die Lösung filtriert und eingeengt.
Ausbeute : 22,8 g 4-Formyl-1-tosyl-indol, F.p. : 140-145°C

B. 22,6 g (86,7 mMol) Chlortitantriisopropoxid werden in 50 ml Äther unter Stickstoff bei −78°C unter Ausschluß von Luftfeuchtigkeit suspendiert. 86,7 mMol Methylmagnesiumjodid in 90 ml Ether werden tropfenweise hinzugegeben. Die resultierende gelbe Lösung wird 5 Minuten bei −78°C gerührt und anschließend tropfenweise mit 20 g (66,9 mMol) 4-Formyl-1-tosylindol in 150 ml absol. THF versetzt. Nach 30 Minuten bei −78°C wurde noch 75 Minuten bei Raumtemperatur gerührt. Danach wurden nacheinander 25 ml gesättigte Ammoniumfluorid-Lösung und 250 ml gesättigte Kochsalzlösung vorsichtig hinzugegeben. Die org. Phase wurde abgetrennt und die wässrige Phase zweimal mit 200 ml Essigester extrahiert. Die vereinigten org. Phasen wurden getrocknet, filtriert und eingeengt. Der Rückstand wurde mit Diisopropyläther ausgerührt.
Ausbeute : 15 g 4-(1-Hydroxyethyl)-1-tosylindol, F.p. : 78-80°C

C. 11,9 g (37,8 mMol) 4-(1-Hydroxyethyl)-1-tosylindol in 150 ml Methylenchlorid wurden bei Raumtemperatur unter Angon mit 13,3 g (85 mMol) Ethyljodid 6,7 g Tetrabutylammoniumhydrogensulfat und 6,7 g (119,3 mMol) gepulvertem Kaliumhydroxid nacheinander versetzt. Die Lösung wurde 2 Stunden gerührt. Nach Filtration über Kieselgur wurde die Lösung erneut mit den gleichen Mengen von Ethyljodid, Tetrabutylammoniumhydrogensulfat und Kaliumhydrogenoxid versetzt. Die Reaktionsmischung wurde 2 Stunden bei Raumtemperatur gerührt, mit 200 ml Wasser gewaschen und die org. Phase getrocknet, filtriert und eingeengt. Anschließende Chromatographie an Kieselgel mit Cyclohexan/Essigester (8 : 2) lieferte 8,5 g 4-(1-Ethoxyethyl)-1-tosylindol als Öl.

D. 3,75 g (10,9 mMol) 4-(1-Ethoxyethyl)-1-tosylindol in 15 ml Ethanol suspendiert wurden zu einer frisch hergestellten Lösung von 628 mg (27 mMol) Natrium in 25 ml Ethanol gegeben und die Mischung 1,5 Stunden zum Rückfluß erhitzt. Nach Verdampfung des Lösungsmittels wurde der Rückstand in 50 ml Wasser aufgenommen und dreimal mit Essigester extrahiert. Die vereinigten org. Phasen wurden mit Wasser gewaschen, getrocknet, filtriert und eingeengt.
Ausbeute : 2 g 4-(1-Äthoxyäthylindol) ; wurde ohne weitere Reinigung für die anschließende Reaktion verwendet.

E. Eine Lösung von 15 g Acetaldehydisopropylimin in 5 ml Toluol wurde bei 10°C zu einer Lösung von 2g 4-(1-Ethoxyethylindol) in 10 ml Eisessig innerhalb von 30 Minuten gegeben. Nach 36 Stunden bei 0-5°C wurde die Lösung in 50 ml Eiswasser eingerührt. Die Mischung wurde mit Toluol extrahiert und die wässrige Phase bei Eiskühlung auf pH 12 mit 2n-Natronlauge eingestellt. Die Lösung wurde mit Äther extrahiert, mit halbgesättigter Kocksalzlösung gewaschen und das Lösungsmittel im Vakuum verdampft. Das Rohprodukt (2,8 g) wurde ohne weitere Reinigung für die anschließende Reaktion verwendet.

F. 2,8 g Amin aus der Reaktion E in 150 ml Toluol und 1,3 ml Nitroessigsäureäthylester wurden 4 Stunden bei 80°C unter Stickstoff gehalten. Nach dem Erkalten wurde mit 0,1N-HCl und Wasser gewaschen. Das Lösungsmittel wurde abdestilliert und das Rohprodukt (3,3 g) an Kieselgel mit Hexan/Essigester chromatographiert.
Ausbeute : 2,7 g 4-(1-Ethoxyethyl)-indol-3-(2-nitro-3-methyl)-propionsäureäthylester als Öl.

G. 2,65 g (7,6 mMol) 4-(1-Ethoxyethyl)-indol-3-(2-nitro-3-methyl)-propionsäureäthylester wurden bei Raumtemperatur und Normaldruck für 1,25 Stunden in 140 ml Ethanol mit 4 ml Raney Nickel (Typ B 115 Z, Fa. Degussa) hydriert. Anschließend wurde die Lösung filtriert und eingeengt.
Ausbeute : 2,3 g 4-(1-Ethoxyethyl)-indol-3-(2-amino-3-methyl)-propionsäureethylester als Öl.

H. 2,17 g (6,82 mMol) 4-(1-Ethoxyethyl)-indol-3-(2-amino-3-methyl)-propionsäureäthylester wurde in 30 ml Xylol gelöst und zu 231 mg suspendiertem Paraformaldehyd in 30 ml Xylol gegeben. Die Reaktionsmischung wurde 75 Minuten am Rückfluß gekocht. Danach wurde eingeengt und an Kieselgel mit Methylenchlorid/Ethanol (10 : 1) chromatographiert.
Ausbeute : 1,35 g 5-(1-Ethoxyethyl)-4-methyl-1,2,3,4-tetrahydro-β-carbolin-3-carbonsäureethylester als Öl.

I. 2,25 g (6,8 mMol) 5-(1-Ethoxyethyl)-4-methyl-1,2,3,4-tetrahydro-β-carbolin-3-carbonsäureethylester in 20 ml Dimethylsulfoxid wurden mit 437 mg Schwefel unter Argon bei 140°C Badtemperatur 30 Minuten gerührt. Nach dem Einengen wurde der ölige Rückstand an Kieselgel zunächst mit Hexan/Aceton (1 : 1) und anschließend mit Methylenchlorid/Ethanol (10 : 1) chromatographiert.
Ausbeute : 426 mg 5-(1-Ethoxyethyl)-4-methyl-β-carbolin-3-carbonsäureetylester, F.p. :159-160°C

Analog wurden hergestellt :
5-(1-Methoxyethyl)-4-methyl-β-carbolin-3-carbonsäureethylester F.p. : 161-163°C
5-(1-Propoxyethyl)-4-methyl-β-carbolin-3-carbonsäureethylester F.p. : 155-156°C
5-(1-Ethoxypropyl)-4-methyl-β-carbolin-3-carbonsäureethylester F.p. : 185-186°C
5-(1-Ethoxyäthyl)-4-methoxymethyl-β-carbolin-3-carbonsäureethylester F.p. : 144-148°C
5-(1-Propoxyethyl)-4-methoxymethyl-β-carbolin-3-carbonsäureethylester

5-(1-Methoxyethyl)-4-ethyl-β-carbolin-3-carbonsäureethylester F.p. : 127-130°C

BEISPIEL 2

5-(Ethoxyethyl)-4-methyl-β-carbolin-3-carbonsäureisopropylester

163 mg 5-(1-Ethoxyethyl)-4-methyl-3-carbonsäureethylester wurden in 5 ml absolutem Isopropanol mit 71 mg Titan (IV) isopropoxid unter Argon und Feuchtigkeitsausschluß 1 Stunde am Rückfluß gekocht. Nach Einengen unter Vakuum wurde der Rückstand in 15 ml 2n-HCl und 15 ml Essigester verteilt. Die wässrige Phase wurde nochmals mit Essigester ausgeschüttelt und mit Ammoniak alkalisch gestellt wobei Titandioxid ausfiel. Auch diese Phase wurde zweimal mit je 15 ml Essigester extrahiert. Die organischen Phasen wurden alle zusammengefaßt eingeengt und über Kieselgel mit Aceton/Hexan (1 : 1) chromatographiert.
Ausbeute : 66 mg 5-(1-Ethoxyethyl)-4-methyl-β-carbolin-3-carbonsäureisopropylester

In analoger Weise wurden folgende Verbindungen hergestellt :
5-(1-Methoxyethyl)-4-methyl-β-carbolin-3-carbonsäureisopropylester
5-(1-Propoxyethyl)-4-methyl-β-carbolin-3-carbonsäureisopropylester F.p. : 153-154°C
5-(1-Propoxyethyl)-4-methoxymethyl-β-carbolin-3-carbonsäureisopropylester
6-(1-Propoxyethyl)-4-methyl-β-carbolin-3-carbonsäureisopropylester
6-(1-Propoxyethyl)-4-methoxymethyl-β-carbolin-3-carbonsäureisopropylester

BEISPIEL 3

5-(1-Ethoxyethyl)-4-methyl-β-carbolin-3-carbonsäure-tert.-butylester

200 mg 5-(1-Ethoxyethyl)-4-methyl-β-carbolin-3-carbonsäure wurden in 4 ml Aminalester 1,5 Stunden auf 120°C Badetemperatur unter Argon und Feuchtigkeitsausschluß erwärmt. Nach Einengen wurde der Rückstand über Kieselgel mit Methylenchlorid/Ethanol (10 : 1) chromatographiert.
Ausbeute : 100 mg 5-(1-Ethoxyethyl)-4-methyl-β-carbolin-3-carbonsäure-tert.-butylester als Öl

In analoger Weise wurden hergestellt :
6-(1-Buthoxyethyl)-β-carbolin-3-carbonsäure-tert.-butylester, F.p. : 189-191°C

BEISPIEL 4

6-(1-Hydroxyethyl)-β-carbolin-3-carbonsäureethylester

A. 6-Acethyl-β-carbolin-3-carbonsäureethylester

400 mg $ALCl_3$ wurden in 10 ml Acetylchlorid bei 0°C suspendiert und 600 mg β-Carbolin-3-carbonsäureethylester wurden hinzugegeben. Nach 5 Minuten wurden 600 mg $ALCl_3$ hinzugegeben und die Mischung 1 Stunde gerührt. Alkohol wurde hinzugegeben und der pH-Wert auf 7-8 eingestellt. 50 ml Wasser wurden hinzugegeben und die filtrierte Mischung ergab 450 mg Rohprodukt, das aus Ethanol/Wasser (1 : 1) umkristallisiert wurde. F.p. : 290-305°C

Analog werden hergestellt :
6-Benzoyl-β-carbolin-3-carbonsäureethylester
6-Acetyl-4-ethyl-β-carbolin-3-carbonsäureethylester
6-(4-Fluorbenzoyl)-β-carbolin-3-carbonsäureethylester
6-(2-Fluorbenzoyl)-β-carbolin-3-carbonsäureethylester

B. 6-(1-Hydroxyethyl)-β-carbolin-3-carbonsäureethylester

250 mg des Produktes von A und 150 mg $NoBH_4$ wurden in 30 ml Methanol für 2 Stunden gerührt und danach 30 Minuten am Rückfluß gekocht. Dann wurden 30 ml Wasser hinzugegeben und das Lösungsmittel verdampft. Man erhielt 22O mg Öl, das über Nacht kristallisiert. F.p. : 125-130°C

Die folgenden Verbindungen wurden analog hergestellt : 4-Methyl-6-(1-hydroxyethyl)-β-carbolin-3-carbonsäureethylester
6-(1-Hydroxybenzyl)-β-carbolin-3-carbonsäureethylester
6-(1-Hydroxyethyl)-4-ethyl-β-carbolin-3-carbonsäureethylester
6-(1-Hydroxy-o-fluorbenzyl)-β-carbolin-3-carbonsäureethylester F.p. 116-117°C

6-(1-Hydroxy-p-fluorbenzyl)-β-carbolin-3-carbonsäureethylester F.p. 212°C

BEISPIEL 5

4-Methyl-6-(1-methoxyethyl)-β-carbolin-3-carbonsäureethylester

100 mg 4-Methyl-6-(1-hydroxyethyl)-β-carbolin-3-carbonsäureethylester wurden mit 10 ml Thionylchlorid bei 0°C für 30 Minuten gerührt. Das Produkt wurde durch Zugabe von 50 ml Petroläther ausgefällt. Die ausgefallenen Kristalle wurden sofort in 10 ml Methanol gelöst und die Lösung 2 Stunden bei Raumtemperatur stehengelassen.

Das Produkt wurde dann ausgefällt durch Zugabe von 20 ml gesättigter Natriumhydrogencarbonat-Lösung. Nach Absaugen des Produktes und Waschen mit Wasser erhielt man 50 mg vom F.p. : 96-97°C.

Die folgenden Verbindungen wurden in analoger Weise hergestellt :

4-Methyl-6-(1-ethoxyethyl)-β-carbolin-3-carbonsäureethylester F.p. : 90-91°C
4-Methyl-6-(1-(2,2,2-trifluorethoxy)-ethyl-β-carbolin-3-carbonsäureethylester F.p. : 164-165°C
4-Methyl-6-(1-isopropoxyethyl)-β-carbolin-3-carbonsäureethylester F.p. : 86-86,5°C
4-Methyl-6-(1-äthylthioethyl)-β-carbolin-3-carbonsäureethylester F.p. : 171-175°C
4-Ethyl-6-(1-ethoxyethyl)-β-carbolin-3-carbonsäureethylester
6-(1-Butoxyethyl)-β-carbolin-3-carbonsäureethylester
6-(1-Propoxyethyl)-β-carbolin-3-carbonsäureethylester
6-(1-Propoxyethyl)-4-methyl-β-carbolin-3-carbonsäureethylester
6-(1-Propoxyethyl)-4-methoxymethyl-β-carbolin-3-carbonsäureethylester
6-(1-Methoxyethyl)-β-carbolin-3-carbonsäureethylester F.p. 189-190°C
6-(1-Phenoxyethyl)-β-carbolin-3-carbonsäureethylester

BEISPIEL 6

3-[5-(3-Ethyl-1,2,4-oxadiazol)-yl]-5-(1-methoxyethyl)-4-methyl-β-carbolin

500 mg 5-(1-Methoxymethyl)-4-methyl-β-carbolin-3-carbonsäure wurden in 20 ml abs. Dimethylformamid bei Raumtemperatur mit 500 mg Carbonyldiimidazol versetzt und 4 Stunden bei dieser Temperatur gerührt. Anschließend wurden 600 mg Propionamidoxim zugegeben und über Nacht bei Raumtemperatur gerührt. Nach Abdestillieren des Lösungsmittels wurde in 50 ml Toluol aufgenommen und 4 Stunden am Wasserabscheider gekocht. Nach Verdünnen mit Essigester wurde nacheinander mit Wasser und gesättigter Kochsalzlösung ausgeschüttelt, getrocknet, filtriert und eingeengt. Der Rückstand wurde über Kieselgel mit Hexan/Aceton (1 : 1) als Elutionsmittel chromatographiert. Man erhielt 250 mg vom F.p. : 213-214°C.

Analog wurden die folgenden Verbindungen hergestellt :

3-[5-(3-Ethyl-1,2,4-oxadiazol)-yl]-6-(1-hydroxyethyl)-β-carbolin F.p. : 220-231°C
3-[5-(3-Ethyl-1,2,4-oxadiazol)-yl]-4-methyl-6-(1-hydroxyethyl)-β-carbolin F.p. : 263-267°C
3-[5-(3-Ethyl-1,2,4-oxadiazol)-yl]-4-methyl-6-(1-methoxyethyl)-β-carbolin F.p. : 255-265°C
3-[5-(3-Ethyl-1,2,4-oxadiazol)-yl]-4-methyl-6-(1-ethoxyethyl)-β-carbolin F.p. : 172-176°C
3-[5-(3-Ethyl-1,2,4-oxadiazol)-yl]-4-methyl-6-(1-(2,2,2-trifluorethoxy) ethyl)-β-carbolin F.p. : 140-155°C
3-[5-(3-Ethyl-1,2,4-oxadiazol)-yl]-4-ethyl-6-(1-ethoxy)-β-carbolin F.p. : 192-195°C
3-[5-(3-Ethyl-1,2,4-oxadiazol)-yl]-4-methyl-5-(1-ethoxyethyl)-β-carbolin F.p. : 180-184°C
3-[5-(3-Ethyl-1,2,4-oxadiazol)-yl]-6-(1-butoxyethyl)-β-carbolin F.p. : 220-226°C
3-[5-(3-Ethyl-1,2,4-oxadiazol)-yl]-4-methyl-5-(1-ethoxypropyl)-β-carbolin F.p. : 202-204°C
3-[5-(3-Ethyl-1,2,4-oxadiazol)-yl]-4-methoxymethyl-5-(1-ethoxyethyl)-β-carbolin F.p. : 175-178°C
3-[5-(3-Ethyl-1,2,4-oxadiazol)-yl]-6-(1-propoxyethyl)-β-carbolin F.p. : 225°C
3-[5-(3-Ethyl-1,2,4-oxadiazol)-yl]-4-methyl-6-(1-isopropoxyethyl)-β-carbolin F.p. : 179-184°C
3-[5-(3-Ethyl-1,2,4-oxadiazol)-yl]-4-methyl-6-(1-ethylthioethyl)-β-carbolin F.p. : 165-170°C
5-(1-Methoxyethyl)-4-ethyl-3-[5-(3-ethyl-1,2,4-oxadiazol)-yl]-β-carbolin F.p. 195-202°C
6-(1-Methoxyethyl)-3-[5-(3-ethyl-1,2,4-oxadiazol)-yl]-β-carbolin F.p. 137-141°C
6-(1-Phenoxyethyl)-4-methyl-3-[5-(3-ethyl-1,2,4-oxadiazol)-yl]-β-carbolin F.p. 150-160°C
6-(1-Hydroxybenzyl)-3-[5-(3-ethyl-1,2,4-oxadiazol)-yl]-β-carbolin F.p. 124°C
6-(1-Hydroxyethyl)-4-ethyl-3-[5-(3-ethyl-1,2,4-oxadiazol)-yl]-β-carbolin F.p. 174-177°C
5-(1-Ethoxypropyl)-4-methoxymethyl-3-(3-ethyl-1,2,4-oxadiazol-5-yl)-β-carbolin

## Ansprüche

1. Verbindungen der allgemeinen Formel I

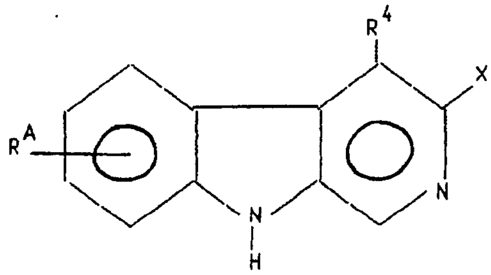

(I)

worin

X eine $COOR^3$-Gruppe mit $R^3$ in der Bedeutung von $C_{1-6}$-Alkyl oder einen Oxadiazolylrest der Formel

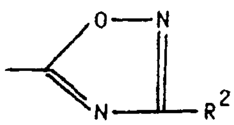

oder

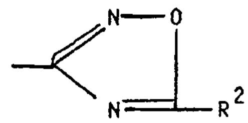

mit $R^2$ in der Bedeutung von H, $C_{1-6}$-Alkyl oder $C_{3-7}$-Cycloalkyl darstellt und

$R^4$ Wasserstoff, $C_{1-6}$-Alkyl oder $C_{1-6}$-Alkoxyalkyl bedeutet und

$R^A$ eine CH $R^1ZR^5$-Gruppe ist, worin Z Schwefel oder Sauerstoff bedeutet, $R^1$ $C_{1-6}$-Alkyl oder gegebenenfalls mit Halogen, $C_{1-6}$-Alkyl oder $C_{1-6}$-Alkoxy substituiertes Phenyl ist und

$R^5$ Wasserstoff, Phenyl oder gegebenenfalls mit Halogen, Phenyl oder $C_{1-2}$-Alkoxy substituiertes Alkyl ist und jede Verbindung 1 oder 2 $R^A$-Reste enthalten kann,

wobei $R^3$ nicht Ethyl ist, wenn $R^4$ $CH_3$ und $R^A$ 5-$CH(OC_2H_5)C_2H_5$ und wenn $R^4$ $CH_2OCH_3$ und $R^A$ 5-$CH(OC_2H_5)$-$CH_3$ bedeuten.

2. 5-(1-Ethoxyethyl)-4-methyl-β-carbolin-3-carbonsäureethylester
5-(1-Methoxyethyl)-4-methyl-β-carbolin-3-carbonsäureethylester
5-(1-Propoxyethyl)-4-methyl-β-carbolin-3-carbonsäureethylester
6-(1-Hydroxyethyl)-β-carbolin-3-carbonsäureethylester
6-(1-Methoxyethyl)-4-methyl-β-carbolin-3-carbonsäureethylester
6-(1-Ethoxyethyl)-4-methyl-β-carbolin-3-carbonsäureethylester
6-[1-(2,2,2-trifluorethoxy)-ethyl]-4-methyl-β-carbolin-3-carbonsäureethylester
6-(1-Ethylthioethyl)-4-methyl-β-carbolin-3-carbonsäureethylester

3. 5-(1-Propoxyethyl)-4-methyl-β-carbolin-3-carbonsäureisopropylester
5-(1-Ethoxyethyl)-4-methyl-β-carbolin-3-carbonsäure-tert.-butylester
6-(1-Butoxyethyl)-β-carbolin-3-carbonsäure-tert.-butylester

4. 6-(1-Ethoxyethyl)-4-methyl-3-(3-ethyl-1,2,4-oxadiazol-5-yl)-β-carbolin
6-(1-Ethoxyethyl)-4-ethyl-3-(3-ethyl-1,2,4-oxadiazol-5-yl)-β-carbolin
5-(1-Ethoxypropyl)-4-methoxymethyl-3-(3-ethyl-1,2,4-oxadiazol-5-yl)-β-carbolin
5-(1-Ethoxypropyl)-4-methyl-3-(3-ethyl-1,2,4-oxadiazol-5-yl)-β-carbolin
5-(1-Ethoxyethyl)-4-methyl-3-(3-ethyl-1,2,4-oxadiazol-5-yl)-β-carbolin

5. Pharmazeutische Präparation enthaltend mindestens eine Verbindung gemäß Anspruch 1-4.

6. Verfahren zur Herstellung der Verbindungen der allgemeinen Formel I, dadurch gekennzeichnet, daß man

a) eine Verbindung der allgemeinen Formel II

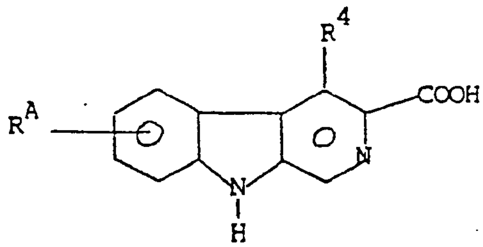

(II)

worin $R^A$ und $R^4$ die oben genannte Bedeutung haben mit einer Verbindung der Formel

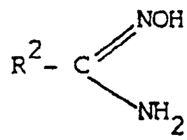

worin $R^2$ die oben genannte Bedeutung hat, umsetzt zu einer Verbindung der allgemeinen Formel I, in der X für den Rest

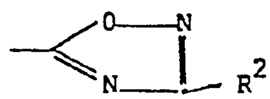

mit $R^2$ in der oben genannten Bedeutung steht,
b) eine Verbindung der allgemeinen Formel III

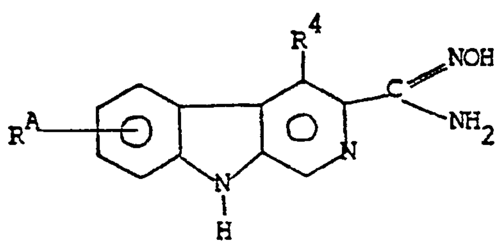

(III),

worin $R^A$ und $R^4$ die oben genannte Bedeutung haben, mit einem Carbonsäureanhydrid $(R^2CO)_2$ 0, worin $R^2$ die oben genannte Bedeutung hat, umsetzt zu einer Verbindung der allgemeinen Formel I, in der X für den Rest

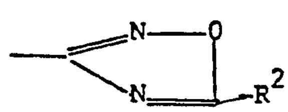

mit $R^2$ in der oben genannten Beudeutung steht,
c) eine Verbindung der allgemeinen Formel IV

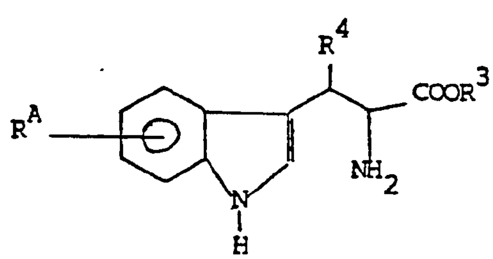

(IV)

worin
$R^4$ die oben genannte Bedeutung hat,
$R^A$ Wasserstoff oder die oben genannten Reste Bedeutet und
$R^3$ Methyl oder Ethyl ist,
cyclisiert und aromatisiert ur.d, falls $R^A$ Wasserstoff ist, acyliert zum 6-Acyl-Derivat und dieses reduziert zu einer Verbindung der allgemeinen Formel I mit $R^A$ in der Bedeutung von $CHR^1$ OH, worin $R^1$ die obige Bedeutung hat und anschließend gegebenenfalls eine freie Hydroxygruppe verethert und/ oder eine Estergruppe umestert.

## Claims

1. Compounds of the general formula I

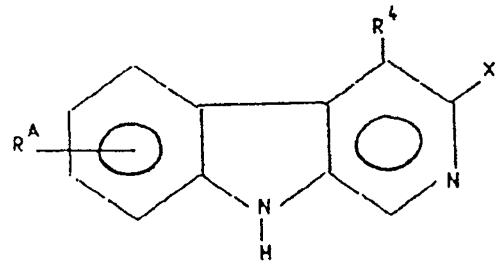

(I)

wherein
X is a $COOR^3$ group, wherein $R^3$ is $C_{1-6}$ -alkyl, or is an oxadiazolyl radical of the formula

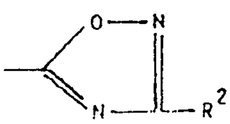

or

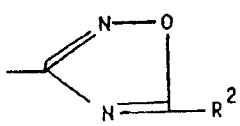

wherein $R^2$ is H, $C_{1-6}$ -alkyl or $C_{3-7}$ -cycloalkyl, and
$R^4$ is hydrogen, $C_{1-6}$-alkyl or $C_{1-6}$-alkoxyalkyl and
$R^A$ is a CH $R^1ZR^5$ group, wherein Z is sulphur or oxygen, $R^1$ is $C_{1-6}$-alkyl, or phenyl optionally substituted by halogen, $C_{1-6}$ -alkyl or by $C_{1-6}$ -alkoxy, and $R^5$ is hydrogen, phenyl, or alkyl optionally substituted by halogen, phenyl or by $C_{1-2}$-alkoxy, and wherein each compound may contain 1 or 2 $R^A$ radicals, and wherein $R^3$ is not ethyl when $R^4$ is $CH_3$ and $R^A$ is 5-$CH(OC_2H_5)C_2H_5$ and when $R^4$ is $CH_2OCH_3$ and $R^A$ is 5-$CH(OC_2H_5)$-$CH_3$.

2. 5-(1-Ethoxyethyl)-4-methyl-β-carboline-3-carboxylic acid ethyl ester
5-(1-Methoxyethyl)-4-methyl-β-carboline-3-carboxylic acid ethyl ester
5-(1-Propoxyethyl)-4-methyl-β-carboline-3-carboxylic acid ethyl ester
6-(1-Hydroxyethyl)-β-carboline-3-carboxylic acid ethyl ester
6-(1-Methoxyethyl)-4-methyl-β-carboline-3-carboxylic acid ethyl ester
6-(1-Ethoxyethyl)-4-methyl-β-carboline-3-carboxylic acid ethyl ester
6-[1-(2,2,2-trifluoroethoxy)-ethyl]-4-methyl-β-carboline-3-carboxylic acid ethyl ester
6-(1-Ethylthioethyl)-4-methyl-β-carboline-3-carboxylic acid ethyl ester.

3. 5-(1-Propoxyethyl)-4-methyl-β-carboline-3-carboxylic acid isopropyl ester
5-(1-Ethoxyethyl)-4-methyl-β-carboline-3-carboxylic acid tert.-butyl ester
6-(1-Butoxyethyl)-β-carboline-3-carboxylic acid tert.-butyl ester.

4. 6-(1-Ethoxyethyl)-4-methyl-3-(3-ethyl-1,2,4-oxadiazol-5-yl)-β-carboline
6-(1-Ethoxyethyl)-4-ethyl-3-(3-ethyl-1,2,4-oxadiazol-5-yl)-β-carboline
5-(1-Ethoxypropyl)-4-methoxymethyl-3-(3-ethyl-1,2,4-oxadiazol-5-yl)-β-carboline
5-(1-Ethoxypropyl)-4-methyl-3-(3-ethyl-1,2,4-oxadiazol-5-yl)-β-carboline
5-(1-Ethoxyethyl)-4-methyl-3-(3-ethyl-1,2,4-oxadiazol-5-yl)-β-carboline.

5. Pharmaceutical preparations containing at least one compound according to any one of claims 1 to 4.

6. A process for the preparation of compounds of the general formula I, characterised in that

a) a compound of the general formula II

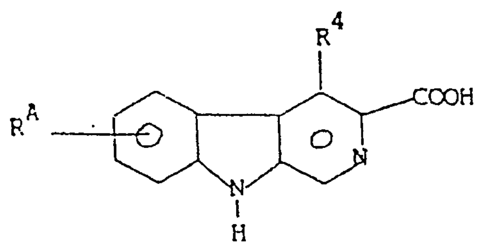

(II),

wherein $R^A$ and $R^4$ have the meanings mentioned above, is reacted with a compound of the formula

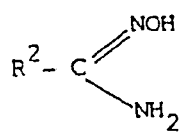

,

wherein $R^2$ has the meaning mentioned above, to form a compound of the general formula I in which X is the radical

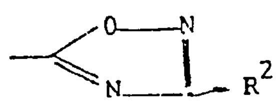

wherein $R^2$ has the meaning mentioned above,
b) a compound of the general formula III

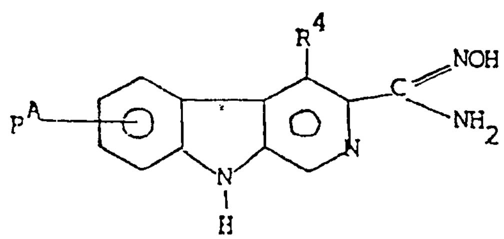

(III),

wherein $R^A$ and $R^4$ have the meanings mentioned above, is reacted with a carboxylic acid anhydride $(R^2CO)_2O$, wherein $R^2$ has the meaning mentioned above, to form a compound of the general formula I in which X is the radical

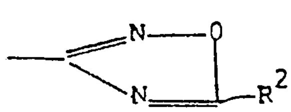

wherein $R^2$ has the meaning mentioned above,
c) a compound of the general formula IV

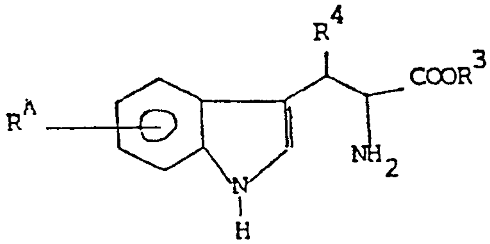

(IV),

wherein
$R^4$ has the meaning mentioned above,
$R^A$ is hydrogen or the radicals mentioned above, and
$R^3$ is methyl or ethyl,
is cyclised and aromatised and, if $R^A$ is hydrogen, is acylated to form the 6-acyl derivative and the latter is reduced to a compound of the general formula I in which $R^A$ is $CHR^1OH$, wherein $R^1$ has the meaning mentioned above, and then optionally a free hydroxy group is etherified and/or an ester group is transesterified.

## Revendications

1. Composés répondant à la formule générale I :

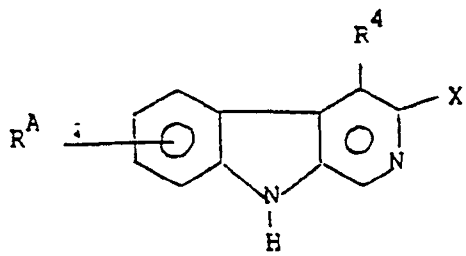

(I)

dans laquelle
X représente un radical $-COOR^3$ dont le symbole $R^3$ représente un alkyle en $C_1$-$C_6$ ou un radical oxadiazolyle répondant à l'une des formules suivantes :

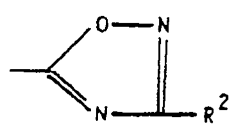

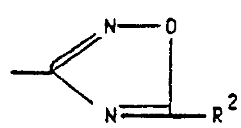

dans lesquelles $R^2$ représente H, un alkyle en $C_1$-$C_6$ ou ou un cycloalkyle et $C_3$-$C_7$,
$R^4$ représente l'hydrogène, un alkyle en $C_1$-$C_6$ ou un alcoxyalkyle en $C_1$-$C_6$, et
$R^A$ représente un radical $-CH(R^1)ZR^5$ dans lequel
Z représente le soufre ou l'oxygène,
$R^1$ représente un alkyle en $C_1$-$C_6$ ou un phényle éventuellement porteur d'un halogène, d'un alkyle en $C_1$-$C_6$ ou d'un alcoxy en $C_1$-$C_6$, et
$R^5$ représente l'hydrogène, un phényle ou un alkyle éventuellement porteur d'un halogène, d'un phényle ou d'un alcoxy en $C_1$ ou $C_2$,
chaque composé pouvant contenir 1 ou 2 radicaux $R^A$, avec les restrictions que $R^3$ ne peut représenter un radical éthyle lorsque $R^4$ représente $CH_3$ et $R^A$ un radical $-CH(OC_2H_5)C_2H_5$ en 5 et lorsque $R^4$ représente un radical $-CH_2OCH_3$ et $R^A$ un radical $-CH(OC_2H_5)CH_3$ en 5.

2. (Ethoxy-1 éthyl)-5 méthyl-4 β-carboline-carboxylate-3 d'éthyle,
(Méthoxy-1 éthyl)-5 méthyl-4 β-carboline-carboxylate-3 d'éthyle,
(Propoxy-1 éthyl)-5 méthyl-4 β-carboline-carboxylate-3 d'éthyle,
(Hydroxy-1 éthyl)-6 β-carboline-carboxylate-3 d'éthyle,
(Méthoxy-1 éthyl)-6 méthyl-4 β-carboline-carboxylate-3 d'éthyle,
(Ethoxy-1 éthyl)-6 méthyl-4 β-carboline-carboxylate-3 d'éthyle,
[(Trifluoro-2,2,2 éthoxy)-1 éthyl]-6 méthyl-4 β-carboline-carboxylate-3 d'éthyle,
(Ethylthio-1 éthyl)-6 méthyl-4 β-carboline-carboxylate-3 d'éthyle.

3. (Propoxy-1 éthyl)-5 méthyl-4 β-carboline-carboxylate-3 d'isopropyle,
(Ethoxy-1 éthyl)-5 méthyl-4 β-carboline-carboxylate-3 de tert-butyle,
(Butoxy-1 éthyl)-6 β-carboline-carboxylate-3 de tert-butyle.

4. (Ethoxy-1 éthyl)-6 méthyl-4 (éthyl-3 oxadiazole-1,2,4 yl-5)-3 β-carboline,
(Ethoxy-1 éthyl)-6 éthyl-4 (éthyl-3 oxadiazole-1,2,4 yl-5)-3 β-carboline,
(Ethoxy-1 propyl)-5 méthoxyméthyl-4 (éthyl-3 oxadiazole-1,2,4 yl-5)-3 β-carboline,

(Ethoxy-1 propyl)-5 méthyl-4 (éthyl-3 oxadiazole-1,2,4 yl-5)-3 β-carboline,
(Ethoxy-1 éthyl)-5 méthyl-4 (éthyl-3 oxadiazole-1,2,4 yl-5)-3 β-carboline.

5. Préparation pharmaceutique comprenant un ou plusieurs composés des deux revendications 1 à 4.

6. Procédé de préparation des composés de formule I de la revendication 1, caractérisé en ce que :

a) on fait réagir un composé de formule II

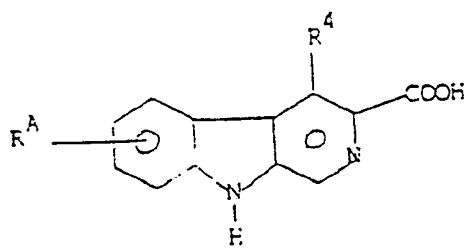

(II)

avec un composé de formule

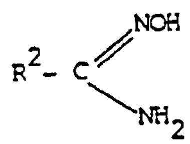

pour former un composé de formule I dans lequel X est le radical

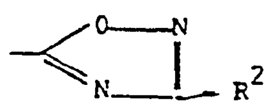

b) on fait réagir un composé de formule III

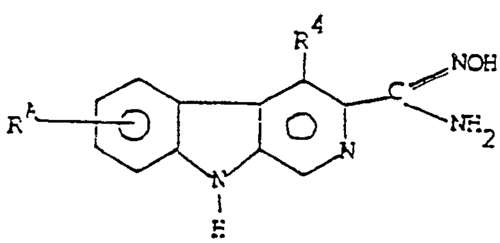

(III)

avec un anhydride d'acide carboxylique $(R^2CO)_2O$ pour former un composé de formule I dans lequel X est le radical ou bien

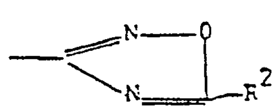

c) on cyclise et aromatise un composé de formule IV

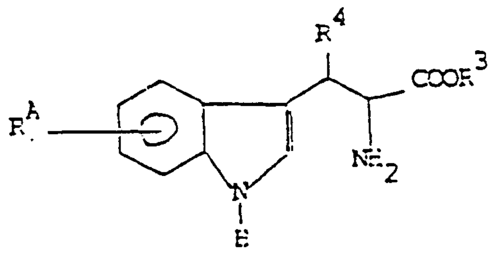

(IV)

$R^A$ Désignant l'hydrogène ou les radicaux ci-dessus indiqués et $R^3$ le groupe méthyle ou éthyle, et si $R^A$ est l'hydrogène on acyle ce composé en un dérivé acylé en 6, que l'on réduit ensuite en un composé de formule I où $R^A$ est un groupe $CHR^1OH$, puis le cas échéant on éthérifie un hydroxyle libre et/ou on transestérifie un groupe ester.